# EUROPEAN PATENT APPLICATION

(11) **EP 4 446 419 A1**
(43) Date of publication of application: **16.10.2024**
(21) Application number: 22904189.2
(22) Date of filing: 05.12.2022
(51) Int. Cl.: C12N 15/864, A61K 35/76, A61P 25/28, C12Q 1/6874, C12Q 1/6883, G01N 33/53

(54) **RECOMBINANT ADENO-ASSOCIATED VIRUS VECTOR FOR TREATMENT OF IRON-ACCUMULATING NEURODEGENERATIVE DISEASES**

(30) Priority: 06.12.2021 JP 2021197789
(71) Applicant: Jichi Medical University, Tochigi 329-0498 (JP)
(72) Inventor: MURAMATSU Kazuhiro, Shimotsuke-shi, Tochigi 329-0498 (JP); TSUKIDA Kiwako, Shimotsuke-shi, Tochigi 329-0498 (JP); MURAMATSU Shin-ichi, Shimotsuke-shi, Tochigi 329-0498 (JP); YAMAGATA Takanori, Shimotsuke-shi, Tochigi 329-0498 (JP)
(74) Representative: J A Kemp LLP
(86) International application number: PCT/JP2022/044719
(87) International publication number: WO 2023/106261

(57) **Abstract**

The present invention provides a novel means of gene therapy for neurodegenerative diseases caused by an iron metabolic disorder. Specifically, the present invention provides a recombinant adeno-associated virus (rAAV) vector which comprises a polynucleotide encoding WDR45 and improves the intracellular expression level of NCOA4. The rAAV vector according to the present invention is useful for the treatment of iron-accumulating neurodegenerative diseases such as SENDA. Also, the present invention provides a method for identifying cells originating in a patient with a neurodegenerative disease, and a method for screening a substance which improves the intracellular expression level of NCOA4 for the treatment of a neurodegenerative disease.

## Description

### TECHNICAL FIELD

The present invention relates to a gene recombinant adeno-associated virus (rAAV) vector for treating iron metabolic disorders in the central nervous system, a pharmaceutical composition comprising the same, and the like.

### BACKGROUND ART

A neurodegenerative disease with brain iron accumulation is a progressive disease characterized by iron accumulation primarily in the basal ganglia, resulting in intellectual disability and motor dysfunction beginning in infancy or adulthood. In addition, this disease is characterized by dystonia or Parkinson-like symptoms, and intellectual regression.

SENDA/BPAN (static encephalopathy of childhood with neurodegeneration in adulthood/Beta-propeller protein-associated neurodegeneration) is known as one type of neurodegeneration with brain iron accumulation (NBIA) that presents with non-progressive intellectual disability beginning in childhood, and with dystonia, Parkinson-like symptoms, and other motor dysfunction and dementia that progress rapidly in adulthood. Based on analysis from patient-derived cells, the cause of SENDA/BPAN (hereafter also referred to as "SENDA") has been reported to involve *de novo* mutations in the WDR45 gene (Non-patent literature 1-4). This gene encodes WDR45 (WIPI4), which is important for autophagy, i.e., an intracellular degradation mechanism essential in organisms (living bodies). Though analysis of central nervous system-specific WDR45 knockout mice showed neural findings such as swelling of the neuronal axons and reduction in the neurons, no clear evidence of iron accumulation (Non-patent literature 2, 5, and 6).

Thus, there is a need to elucidate the mechanism of intracellular iron metabolism in the central nervous system and to provide a means to treat an iron-accumulating neurodegenerative disease caused by this mechanism.

### CITATION LIST

### Non-patent literature

Non-patent literature 1: Saitsu H, et al., Nat. Genet 2013; 45: 445-9
Non-patent literature 2: Kazuhiro Muramatsu, NO TO HATTATSU (Official Journal of the Japanese Society of Child Neurology), 2016; 48: 177-83
Non-patent literature 3: Kiwako Tsukida et al., Clinical Neuroscience 2019; 37: 291-294
Non-patent literature 4: Kazuhiro Muramatsu et al., The Medical Frontline 2017; 72(2): 193-204
Non-patent literature 5: Zhao Y. G., et al., Autophagy 2015; 11: 881-90
Non-patent literature 6: Wan H, et al., Autophagy 2020; 16(3): 531-547

### SUMMARY OF INVENTION

### Technical Problem

In neurodegenerative diseases caused by autophagy dysfunction and iron accumulation in the brain, it is inferred that excess iron present in the cells in the central nervous system produces reactive oxygen species, which in turn cause neuronal damage. However, the mechanism of iron accumulation in the cells in the central nervous system, especially in the brain, is not yet understood, and there is no effective treatment for neurodegenerative diseases caused by iron accumulation.

### Solution to Problem

The inventors of the present application further analyzed the expression of various molecules involved in iron metabolism in detail using cells derived from patients with SENDA/BPAN and it was found, commonly in the patients' cells, that iron components in the form of ferritin were accumulated, that the expression level of NCOA4, which is essential for iron metabolism, was greatly reduced, and that this reduction in the expression level of NCOA4 was a factor in the iron accumulation in the cells in the central nervous system.

The present inventors further found that intracellular ferritin degradation was suppressed when NCOA4 was reduced or disappeared, and that this was a factor in the intracellular iron accumulation of this disease.

Based on these findings, the *WDR45* gene was actually expressed in patient-derived cells using an AAV vector to restore the expression level of NCOA4 in the cells and thus correct ferritin accumulation, and thereby the present invention has been achieved.

The present application provides, for example, an adeno-associated virus vector, a pharmaceutical composition comprising the same, and the like described below.
[1] A recombinant adeno-associated virus vector for use in intracellularly expressing any one of the amino acid sequences represented by SEQ ID NOs: 3-6, the recombinant adeno-associated virus vector comprising a polynucleotide encoding the amino acid sequence represented by SEQ ID NO: 1 or 2, or an amino acid sequence having about 90% or more identity to the amino acid sequence represented by SEQ ID NO: 1 or 2, wherein said amino acid sequence preferably forms a virus vector in the same manner as does the wild-type sequence.
[2] The adeno-associated virus vector according to [1] above, which is used to regulate intracellular autophagy capacity and/or iron metabolism capacity.
[3] The adeno-associated virus vector according to [1] above, which is used for the treatment of an iron-accumulating neurodegenerative disease.
[4] The adeno-associated virus vector according to [1] above, which is used for the treatment of SENDA/BPAN (WDR45 abnormality).
[5] The recombinant adeno-associated virus vector according to [1] above, comprising a capsid protein of wild-type AAV1, AAV2, AAV9, or AAVrh10.
[6] The recombinant adeno-associated virus vector according to [1] above, comprising a capsid protein having a mutant amino acid sequence in which tyrosine at position 445 in the amino acid sequence of wild-type AAV1 capsid protein is replaced with phenylalanine, a capsid protein having a mutant amino acid sequence in which tyrosine at position 445 in the amino acid sequence of wild-type AAV2 capsid protein is replaced with phenylalanine, or a capsid protein having a mutant amino acid sequence in which tyrosine at position 446 in the amino acid sequence of wild-type AAV9 capsid protein is replaced with phenylalanine.
[7] The recombinant adeno-associated according to [1] above, wherein the polynucleotide comprises an inverted terminal repeat (ITR) selected from the group consisting of AAV1, AAV2, AAV3, AAV4, AAV8, AAV9, and AAVrh10.
[8] The recombinant adeno-associated virus vector according to [1] above, wherein the polynucleotide comprises a promoter sequence selected from the group consisting of synapsin I promoter sequence, myelin basic protein promoter sequence, neuron-specific enolase promoter sequence, calcium/calmodulin-dependent protein kinase II (CMKII) promoter sequence, αI-tubulin promoter sequence, platelet-derived growth factor β-chain promoter sequence, glial fibrillary acidic protein (GFAP) promoter sequence, L7 promoter sequence (cerebellar Purkinje cell-specific promoter), glial fibrillary acidic protein (hGfa2) promoter sequence, glutamate receptor delta 2 promoter (cerebellar Purkinje cell-specific promoter) sequence, glutamate decarboxylase (GAD65/GAD67) promoter sequence, WDR45 promoter, NCOA4 promoter, cytomegalovirus promoter, and CAG promoter.
[9] The adeno-associated virus vector according to [1] above, further comprising a polynucleotide encoding any one of the amino acid sequences represented by SEQ ID NOs: 3-6 or an amino acid sequence having about 90% or more identity to any one of the amino acid sequences represented by SEQ ID NOs: 3-6.
[10] A pharmaceutical composition comprising the recombinant adeno-associated virus vector according to any one of [1]-[9] above.
[11] A pharmaceutical composition comprising the recombinant adeno-associated virus vector according to any one of [1]-[8] above, and a recombinant adeno-associated virus vector comprising a polynucleotide encoding any one of the amino acid sequences represented by SEQ ID NOS: 3-6 or an amino acid sequence having about 90% or more identity to any one of the amino acid sequences represented by SEQ ID NOS: 3-6.
[12] The pharmaceutical composition according to [10] above, which is administered intracerebrally, intrathecally or peripherally.
[13] A method for identifying, *in vitro* or *ex vivo,* a cell that causes an iron-accumulating neurodegenerative disease in an organism (living body), the method comprising the steps of:
   providing sample cells derived from the organism;
   measuring the expression level of any one of the amino acid sequences represented by SEQ ID NOs: 3-6 or a coding sequence thereof in the sample cells; and
   comparing the measured expression level with the expression level of any one of the amino acid sequences represented by SEQ ID NOs: 3-6 or a coding sequence thereof in a control cell.
[14] The method according to [13] above, wherein the sample cells are selected from the group consisting of oligodendrocytes, neurons, astrocytes, glial cells, lymphoid cells, and dermal fibroblasts.
[15] The method according to [13] above, wherein the sample cells are cultured *in vitro.*
[16] The method according to [13] above, comprising a step of measuring the expression of any one of the amino acid sequences represented by SEQ ID NOs: 3-6 or a coding sequence thereof with an antibody or by RT-PCR.

### Advantageous Effects of Invention

A vector according to the present invention can provide a therapeutic means useful for an iron-accumulating neurodegenerative disease by regulating iron metabolism in the central nervous system. The method of the present invention may also be used to diagnose patients at risk for an iron-accumulating neurodegenerative disease or patients with such a disease.

### BRIEF DESCRIPTION OF DRAWINGS

[Figure 1] Figure 1 is a schematic view of pathways from uptake of iron from outside the cell, via intracellular transport and the like, to the subsequent efflux, in cells with WDR45 disorder and NCOA4 deficiency.
[Figure 2] Figure 2 shows comparison of the expression levels of WDR45, NCOA4, ferritin heavy chain, ferritin light chain, transferrin receptor, Divalent Metal Transporter 1 (DMT1), and ferroportin, between cells from healthy individuals and cells from Patient 1-4.
[Figure 3] Figure 3 shows phenotypic recovery of NCOA4, ferritin heavy chain, ferritin light chain, transferrin receptor, and ferroportin, by gene transfer of WDR45 into the patient derived cells.

### DESCRIPTION OF EMBODIMENTS

The present application provides an rAAV vector for treating an iron-accumulating neurodegenerative disease by restoring iron metabolism to normal level in the central nervous system of an organism to inhibit iron accumulation.

### 1. Intracellular uptake, transport, efflux, etc.

In an organism, the iron content is generally said to be about 60 mg in the adult brain. As to iron contents in various brain regions, extrapyramidal systems, including the globus pallidus, red nucleus, substantia nigra, and putamen contain more iron contents. Among the cells in the central nervous system, the iron content is high in the oligodendrocytes, followed by the neurons and microglia, and low in the astrocytes. Moreover, the iron content in the brain generally increases with age (Clinical Neurology 2012; 52: 943-946).

The functions of iron in the brain are varied. Iron is also contained in the iron-sulfur centers of complexes I-III and cytochromes, which are major components of the mitochondrial electron-transfer system. Iron, in the form of an iron-containing enzyme (or coenzyme), is also involved in maintenance of TCA cycle functions, myelin formation, nucleic acid biosynthesis, and biosynthesis and metabolism of various neurotransmitters.

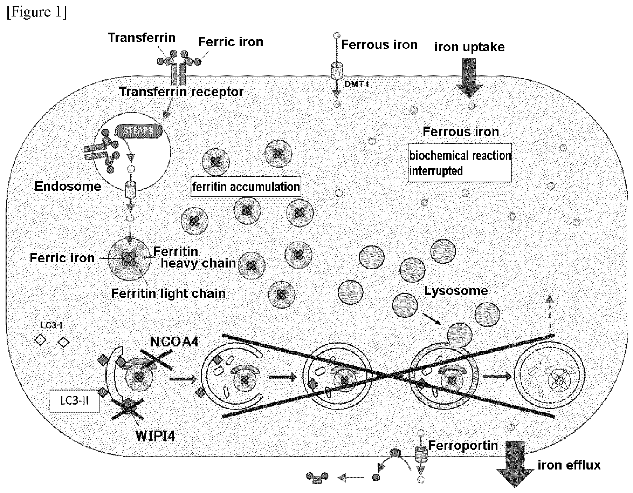

The three main kinetics of iron in the cells of an organism are (1) uptake from the blood, (2) intracellular metabolism, transport, storage, etc., and (3) extracellular efflux. Various factors are known to act in each kinetics (Figure 1) (The Journal of Japanese Biochemical Society 2018; Vol. 90, No. 3: 272-278).

In the blood, ferrous iron ions in the free state and ferric iron ions in the form of holotransferrin bound to an apotransferrin protein or in other form are transported to various sites in the organism. Holotransferrin in blood can be taken up into the cell via transferrin receptor on the cell membrane. Ferrous iron ions can be taken up via DMT1 on the cell membrane.

Transferrin taken up into the cell undergoes endosome-mediated transferrin degradation. Ferric iron released by transferrin degradation are reduced by STEAP3, and ferrous iron ions are released into the cytoplasm.

Further, ferric iron accumulate in ferritin particles in the cell. Ferritin is composed of 24 subunits, which contain two types of proteins, a heavy chain of about 21 kDa and a light chain of about 19 kDa. The ferritin heavy chain oxidizes ferrous iron to less reactive and less toxic ferric iron, and the ferric iron is stored in the ferritin light chain (Biasiotto G, et al., Mol Neurobiol. 2016 Oct; 53(8): 5542-74; Yutaka Kohgo, Takaaki Otake, The Japanese Journal of Surgical Metabolism and Nutrition 2015; 49. 59-65; Kazuhiro Iwai, Ryo Ueta, The Journal of Japanese Biochemical Society 79, 1021-1031, 2007). Ferric iron accumulated in ferritin undergo lysosomal uptake and others.

In addition, ferrous iron can be taken up from outside the cell or can arise from ferric iron during endosomal degradation or are released by ferritin degradation by lysosomes within the cell. This ferrous iron is taken up by mitochondria and plays a role in reduction/oxidation reactions, etc., or is transported to the endoplasmic reticulum.

Iron that is not utilized in the neuron is either stored as ferritin iron in the cell or effluxed from the cell via ferroportin. The effluxed iron binds apotransferrin and travels through the blood in the form of holotransferrin.

### 2. Regarding iron-accumulating neurodegenerative disease

Accumulation of iron in the central nervous system, especially in the brain, is known to cause various diseases. Such diseases are commonly referred to as iron-accumulating neurodegenerative diseases (Neurodegeneration with Brain Iron Accumulation: NBIA). Common neurological syndromes of the diseases include progressive dystonia, ataxia, and parkinsonism, with specific symptoms including gait disturbance, dysarthria, and cognitive impairment (Non-patent literature 2).

NBIA can be classified into two major categories. One is diseases related to iron efflux from the inside to the outside of the cell, iron storage, and the like, and the other is diseases related to lipid metabolism, energy production, and autophagy in neurons.

In general, the process of autophagy involves a step in which a phagophore that appears in the cytoplasm engulfs cytoplasmic materials to form a vesicle (autophagosome), and a step in which the formed autophagosome fuses with a lysosome to degrade the engulfed cytoplasmic materials. The formation of the autophagosomal membrane involves a variety of membrane components derived from the endoplasmic reticulum, mitochondria, Golgi apparatus, plasma membrane, and even recycling endosome, and their formation and the degradation of their contents are regulated by Agt protein encoded by an autophagy-related gene (ATG) and the like (The Journal of Japanese Biochemical Society 2018; Vol. 90, No. 3: 272-278).

SENDA is an example of the autophagy-related neurodegenerative diseases included in NBIA. The pathophysiology of SENDA includes non-progressive intellectual disability beginning in childhood, as well as dystonia, Parkinson-like symptoms, and dementia that progress rapidly in adulthood. Iron accumulation in the substantia nigra and globus pallidus in the brain as well as cerebral atrophy are observed in SENDA patients. The treatment of SENDA include use of a dopamine formulation and intrathecal baclofen therapy (ITB therapy). However, a highly effective treatment has not been established (Non-patent literature 2).

A lymphoblastoid cell line from a patient with SENDA showed significantly decreased WDR45 expression, decreased autophagy activity, and impaired autophagosome formation. In addition, increase in the expressions of Atg9L1 and LC3-II, which are considered markers of autophagy, was also observed (Non-patent literature 2).

### 3. Proteins used in the present invention

### (a) WDR45 (WIPI4)

Genetic analysis, including whole exome analysis, of the SENDA lineage aimed to identify the causative gene of SENDA identified a *de novo* mutation in the WDR45 gene (Non-patent literature 1).

Human WDR45 (WR repeat-containing protein 45) (also referred to herein as "WIPI4") encoded by the gene is a protein that has multiple isoforms, for example, in the case of human, isoform 1 with 361 amino acid residues (GenBank: NP_009006.2) and isoform 2 with 360 amino acid residues (GenBank: NP_001025067.1). WDR45 is one of four human homologs (WIPI1-WIPI4) of the budding yeast autophagy-related gene ATG18. As a more detailed function of ATG18 in budding yeast, it is thought to be involved in the process of autophagosome formation in the cell by binding to intracellularly generated phosphatidylinositol 3-phosphate (PI3P) (Ryo-iki Yugo Review (Reviews in Fused Fields) by Leading Author's 2014; 3, e006, 1-11). It was later reported that it is involved, along with ATG2, in supplying lipids such as phospholipids for the formation of phagophore from the endoplasmic reticulum (Osawa T. et al., Nat Struct Mol Biol 26, 281-288 (2019)).

Examples of the specific nucleotide sequence of WDR45 include GenBank accession number NM_007075.4. The amino acid sequences of human WDR45 used in the present invention are represented by SEQ ID NO: 1 (isoform 1) and SEQ ID NO: 2 (isoform 2) (in which the first methionine residues are excluded).

The expression of WDR45 can be detected by a means known in the art, such as Western blotting using anti-WDR45 antibody (Cat# 19194-1-AP) provided by Proteintech, or RT-PCR using primers appropriately designed based on the coding sequence.

### (b) Nuclear receptor coactivator 4 (NCOA4)

Nuclear receptor coactivator 4 (NCOA4, also called ARA70) has been reported to have, in the case of human, isoform 1 with 650 amino acids (Gene ID: 8031: NM_001145260.2), isoform 2 with 630 amino acids (NP_001138733.1), isoform 3 with 614 amino acids (AAH01562.1, NP_001138734.1, NP_001138735.1, NP_005428.1), one with 575 amino acids (AAH12736.1), or the other. NCOA4 functions as a coactivator of nuclear receptors (androgen receptor, estrogen receptor, glucocorticoid receptor, etc.) (Kollara, A. et al., (2010), J. of Histochemistry and Cytochemistry, 58(7): 595-609, etc.), and as a cargo receptor, which binds to the ferritin heavy chain and recruits ferritin to an autophagosome (Mancias, J. D., et al., (2014), Nature 509 (7498): 105-109, etc.).

A more specific role of NCOA4 in the intracellular iron transport is as follows. Ferritin is engulfed by a phagophore by binding to NCOA4. The phagophore closes to form an autophagosome, and when a lysosome fuses with it, it becomes an autolysosome. When the contents are degraded by hydrolase in the lysosome, the ferric iron encapsulated in ferritin is reduced to ferrous iron and released into the cytoplasm. This ferrous iron is taken up by the intracellular organelles and used for various cellular activities.

Overexpression of the NCOA4 in cells has been reported to promote ferroptosis and cause cell death (Hou, W., et al., AUTOPHAGY 2016, VOL. 12, NO. 8, 1425-1428). Therefore, an optimal NCOA4 expression level is considered to be important for normal intracellular iron metabolism, and appropriate gene transfer of the NCOA4 gene is expected to enable the establishment of a treatment for iron-accumulating neurodegeneration.

Expression of NOCA4 protein can be detected directly by immunostaining such as Western blotting using a commercially available antibody such as anti-ARA70 antibody (#Cat no. A302-272A) provided by Bethyl Laboratories, or can be detected indirectly by RT-PCR (or quantitative RT-PCR (qRT-PCR)), or the like.

### (c) Sequence identity

Heterologous sequences corresponding to the human sequences represented by SEQ ID NOs: 1-6 above can also be used as the amino acid sequence of the protein to be expressed by the vector of the present invention. Examples of such amino acid sequences include those derived from mammals such as mice, rats, monkeys, dogs, pigs, cows, and horses. Preferably, a vector according to the present invention contains a polynucleotide encoding the amino acid sequence represented by SEQ ID NO: 1 or SEQ ID NO: 2, and optionally a polynucleotide encoding any of the amino acid sequences represented by SEQ ID NOs: 3-6 or its corresponding amino acid sequence derived from a non-human animal which has about 90% or more identity to said sequence. More preferably, a vector of the present invention contains a polynucleotide encoding the amino acid sequence represented by SEQ ID NO: 1 or SEQ ID NO: 2, and optionally a polynucleotide encoding any of the amino acid sequences represented by SEQ ID NOs: 3-6.

The protein used in the present invention also includes proteins which have an amino acid sequence with about 85% or more, about 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, and 99.9% or more identity to any of the amino acid sequences represented by SEQ ID NOs: 1-6, and which have the same function as the original protein under a physiological condition. In general, the higher the value above, the better. In the present invention, if a protein with the above identity has the same function as the original protein, it will preferably function to the same extent. According to the present invention, "function to the same extent" means that the specific activity is in the range of, for example, but not limited to, about 0.01-100, preferably about 0.5-20, and more preferably about 0.5-2 under a physiological condition.

Furthermore, the protein used in the present invention includes proteins which contain an amino acid sequence in which one or more amino acids are deleted, substituted, inserted and/or added in any of the amino acid sequences represented by SEQ ID NOS: 1-6 or in an amino acid sequence having the above identity, and which have the same function as the original protein under a physiological condition. Two or more of the above-mentioned amino acid deletions, substitutions, insertions, and additions may occur simultaneously. Examples of such proteins include, for example, proteins which contain an amino acid sequence in which 1-50, 1-40, 1-39, 1-38, 1-37, 1-36, 1-35, 1-34, 1-33, 1-32, 1-31, 1-30, 1-29, 1-28, 1-27, 1-26, 1-25, 1-24, 1-23, 1-22, 1-21, 1-20, 1-19, 1-18, 1-17, 1-16, 1-15, 1-14, 1-13, 1-12, 1-11, 1-10, 1-9 (one to several), 1-8, 1-7, 1-6, 1-5, 1-4, 1-3, 1-2, or 1 amino acid residue in the amino acid sequence represented by SEQ ID NO: 1 or SEQ ID NO: 2 is deleted, substituted, inserted and/or added, and which have the original function under a physiological condition. In general, the smaller the number of the above-mentioned amino acid residues deleted, substituted, inserted and/or added, the better.

Examples of amino acid residues which are replaceable with each other in the protein (polypeptide) of the present invention are listed below. Amino acid residues belonging to the same group are replaceable with each other.
Group A: Leucine, isoleucine, norleucine, valine, norvaline, alanine, 2-aminobutanoic acid, methionine, o-methyl serine, t-butyl glycine, t-butyl alanine, cyclohexylalanine;
Group B: Aspartic acid, glutamic acid, isoaspartic acid, isoglutamic acid, 2-aminoadipic acid, 2-aminosuberic acid;
Group C: Asparagine, glutamine;
Group D: Lysine, arginine, ornithine, 2,4-diaminobutanoic acid, 2,3-diaminopropionic acid;
Group E: Proline, 3-hydroxyproline, 4-hydroxyproline;
Group F: Serine, threonine, homoserine;
Group G: Phenylalanine, tyrosine.

A protein having a substituted amino acid residue can be prepared according to a method known to those skilled in the art, such as a common genetic engineering technique. For such a genetic engineering procedure, refer to, for example, Molecular Cloning 3rd Edition, J. Sambrook et al., Cold Spring Harbor Lab. Press. 2001, Current Protocols in Molecular Biology, John Wiley and Sons 1987-1997, and the like.

In addition, examples of the preferred polynucleotide used in the present invention include polynucleotides which have one or more (e.g., 1-50, 1-40, 1-30, 1-25, 1-20, 1-15, 1-10, 1-9 (one to several), 1-8, 1-7, 1-6, 1-5, 1-4, 1-3, 1-2, 1, etc.) nucleotides deleted, substituted, inserted, and/or added in a polynucleotide sequence encoding any of the amino acid sequences represented by SEQ ID NOs: 1-6, and which encode a protein containing any of the amino acid sequences represented by SEQ ID NOs: 1-6 or which encode a protein containing an amino acid sequence in which one or more of the above-mentioned amino acids in any of the amino acid sequences represented by SEQ ID NOs: 1-6 are deleted, substituted, inserted, and/or added and having the same function as the original protein. A combination of two or more of these deletions, substitutions, insertions, and additions may be contained simultaneously. In general, the smaller the number of the above-mentioned nucleotides for deletion, substitution, insertion, and/or addition, the better. Furthermore, a preferred polynucleotide according to the present invention is, for example, a polynucleotide which can hybridize with a complementary sequence of a polynucleotide encoding any of the amino acid sequences represented by SEQ ID NOs: 1-6 under a stringent hybridization condition, and which encodes any of the amino acid sequences represented by SEQ ID NOs: 1-6 or which encodes a protein containing an amino acid sequence having one or more of the above-mentioned amino acids for deletion, substitution, insertion, and/or addition in any of the amino acid sequences represented by SEQ ID NOs: 1-6 and having the same function as the original (wild-type) protein, preferably a protein that functions to the same extent as described above.

Hybridization can be carried out according to a known method or a method corresponding thereto, for example, according to a method described in Molecular Cloning (Molecular Cloning 3rd Edition, J. Sambrook et al., Cold Spring Harbor Lab. Press. 2001). In a case of using a commercially available library, hybridization can be performed according to, for example, the method described in the instructions provided by the manufacturer. Herein, a "stringent condition" may be any of low, moderate or high stringent conditions. The "low stringent condition" refers to conditions of, for example, 5x SSC, 5x Denhardt's solution, 0.5% SDS, and 50% formamide at 32°C. Alternatively, the "moderate stringent condition" refers to conditions of, for example, 5x SSC, 5x Denhardt's solution, 0.5% SDS, and 50% formamide at 42°C. The "high stringent condition" refers to conditions of, for example, 5x SSC, 5x Denhardt's solution, 0.5% SDS, and 50% formamide at 50°C. Under these conditions, the higher the temperature, the more efficiently DNA with higher homology can be expected to be obtained. Note that multiple factors including the temperature, the probe concentration, the probe length, the ionic strength, the time, the salt concentration, and the like are considered to affect the stringency of hybridization, while those skilled in the art can appropriately select these factors to achieve similar stringency.

Examples of hybridizable polynucleotides include polynucleotides having, for example, 70% or more, 80% or more, 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more identity to a polynucleotide sequence encoding any of the amino acid sequences represented by SEQ ID NOs: 1-6, as calculated by a homology search software such as FASTA and BLAST using default parameters. In general, the higher the homology mentioned above, the better.

The identity or the homology of the amino acid sequence or the polynucleotide sequence can be determined using the BLAST algorithm of Karlin and Altschul (Proc. Natl. Acad. Sci. USA 872264-2268, 1990; Proc. Natl. Acad. Sci USA 90: 5873, 1993). Programs called BLASTN and BLASTX derived from the BLAST algorithm have been developed (Altschul SF, et al., J Mol Biol 215: 403, 1990). If BLASTN is used to analyze the nucleotide sequence, the parameters are, for example, as follows: score = 100, wordlength = 12. Alternatively, if BLASTX is used to analyze the amino acid sequence, the parameters are, for example, as follows: score = 50, wordlength = 3. If BLAST and Gapped BLAST programs are used, the default parameters of each program are used.

### 4. Recombinant adeno-associated virus (rAAV) vector of the present invention

Gene therapies, in which a therapeutic gene is delivered to the nervous system cells, have been investigated for the treatment of various genetic diseases. Examples of the therapeutic vector known in the art include an adenovirus vector, an adeno-associated virus vector, and a lentivirus vector. In addition, an expression vector, such as mRNA, a plasmid, or an artificial chromosome, can also be used directly or in combination with a liposome, or the like.

The present invention primarily uses a recombinant adeno-associated virus (rAAV) vector as a means of delivering a therapeutic gene to nervous system cells. Such rAAVs are described, for example, in International Publications WO2012/057363, WO2008/124724, WO2003/093479, etc.

According to the present invention, the vector for delivering a gene encoding the above-mentioned WDR45 protein and/or a gene encoding NCOA4 as a therapeutic gene to nervous system cells can be a mutant recombinant adeno-associated virus vector described in WO2012/057363, or a recombinant adeno-associated virus vector described in WO2008/124724, etc. The rAAV vector used in the present invention is capable of passing through the blood-brain barrier of an organism, and thus can introduce a desired therapeutic gene into the nervous system cells in a patient's brain, spinal cord, etc. by a means of administration for delivery to the brain through the blood-brain barrier, such as peripheral administration or intrathecal administration to the patient. The rAAV vector used in the present invention is also capable of highly efficient gene transfer when administered directly to or near the target site in the brain.

The rAAV vectors of the present invention can be prepared preferably from, but are not limited to, native adeno-associated virus serotype 1 (AAV1), serotype 2 (AAV2), serotype 3 (AAV3), serotype 4 (AAV4), serotype 5 (AAV5), serotype 6 (AAV6), serotype 7 (AAV7), serotype 8 (AAV8), serotype 9 (AAV9), and serotype AAVrh10. Nucleotide sequences of these adeno-associated viral genomes are known and reference can be made, for example, to the nucleotide sequences registered under GenBank accession numbers: AF063497.1 (AAV1), AF043303 (AAV2), NC_001729 (AAV3), NC_001829.1 (AAV4), NC_006152.1 (AAV5), AF028704.1 (AAV6), NC_006260.1 (AAV7), NC_006261.1 (AAV8), AY530579 (AAV9), and JC105350.1 (AAVrh10) (WO2013174760), respectively. Among these, serotypes 2, 3, 5 and 9 are of human origin. According to the present invention, it is particularly desirable to utilize capsid proteins derived from AAV1, AAV2, AAV9 or AA Vrh10 (VP1, VP2, VP3, etc.). This is due to the fact that, among human-derived AAVs, these AAVs have been reported to have a relatively high efficiency in infecting neurons (Taymans, et al., Hum Gene Ther 18: 195-206, 2007, etc.).

The capsid protein contained in the rAAV vector of the present invention is, preferably as described in WO2012/057363 or WO2008/124724, a mutant protein having an amino acid sequence in which at least one tyrosine is replaced by another amino acid such as phenylalanine compared to its wild-type amino acid sequence. Examples include a mutant protein which has the amino acid sequence in which tyrosine at position 445 in the amino acid sequence of wild-type AAV1 capsid protein is replaced by phenylalanine (SEQ ID NO: 7), the amino acid sequence in which tyrosine at position 444 in the amino acid sequence of wild-type AAV2 capsid protein is replaced by phenylalanine (SEQ ID NO: 8), or the amino acid sequence in which a tyrosine residue at position 446 in the amino acid sequence of wild-type AAV9 capsid protein is replaced by a phenylalanine residue (SEQ ID NO: 9), and which is capable of forming a capsomere or viral vector (WO2012/057363, WO2008/124724, in which the first methionine residues (Met) are deleted). The capsid protein contained in the rAAV vector of the present invention may include proteins which have an amino acid sequence having about 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more identity to any of the above-mentioned amino acid sequences represented by SEQ ID NOs: 7-9, and which are capable of forming a capsomere. In addition, examples include proteins which contain an amino acid sequence in which, for example, 1-50, 1-40, 1-35, 1-30, 1-25, 1-20, 1-15, 1-14, 1-13, 1-12, 1-11, 1-10, 1-9 (one to several), 1-8, 1-7, 1-6, 1-5, 1-4, 1-3, 1-2, or 1 amino acid residue in any of the amino acid sequence represented by SEQ ID NOs: 7-9 is deleted, substituted, inserted and/or added, and which are capable of forming a capsomere. A combination of two or more of these deletions, substitutions, insertions, and additions may be contained simultaneously. In another embodiment, the capsid protein used in the present invention has the function of forming a capsomere alone or with other capsid protein member (e.g., VP2, VP3, etc.). A polynucleotide containing a desired therapeutic gene to be delivered to the nervous system cells, and the like is packaged in the capsomere.

If the rAAV vector according to the present invention is to be administered into the bloodstream, one that can pass through the blood-brain barrier of an organism, including adults and fetuses, can be used. According to the present invention, nervous system cells as the target of gene transfer include at least neurons in the central nervous system, such as the brain and spinal cord, and may also include glial cells, microglia, astrocytes, oligodendrocytes, cerebral ventricular ependymal cells, cerebral vascular endothelial cells, and the like. The percentage of neurons among the nervous system cells to be genetically transduced is preferably 70% or more, 80% or more, 85% or more, 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, 99.9% or more, or 100%.

A Rep protein used in the rAAV vector of the present invention may have a similar percentage of amino acid sequence identity as mentioned above, and may contain a similar number of amino acid residues for deletion, substitution, insertion, and/or addition as mentioned above, as long as it has the known functions, including the function of recognizing ITR sequences and performing genome replication depending on said sequences, the function of recruiting and packaging the wild-type AAV genome (or rAAV genome) into the viral vector, and the function of forming the rAAV vector according to the present invention, to the same extent. The scope of `functionally the same extent' may include the extent that is described in the explanation of the specific activity above. According to the present invention, a Rep protein from a known AAV3 is preferably used.

A polynucleotide encoding the Rep protein used in the preparation of the rAAV vector of the present invention may have a similar percentage of identity as mentioned above, or may contain a similar number of nucleotides for deletion, substitution, insertion, and/or addition as mentioned above, as long as it codes for a Rep protein that has the known functions, including the function of recognizing ITR sequences and performing genome replication in a sequence-dependent manner, the function of packaging the wild-type AAV genome (or rAAV genome) into the viral vector, and the function of forming the rAAV vector of the present invention, to the same extent. The scope of `functionally the same extent' may refer to the extent that is described in the description of the specific activity above. In the present invention, rep gene derived from AAV2 or AAV3 is preferably used.

According to one embodiment of the present invention, the above-described capsid protein VP1, etc. (VP1, VP2 and/or VP3) and Rep protein encoded in the internal domain of the wild-type AAV genome are used by incorporating polynucleotides coding for these proteins into an AAV helper plasmid. If necessary, the capsid protein (VP1, VP2 and/or VP3) and the Rep protein used in the present invention may be incorporating into one, two, three or more of plasmids. In some cases, one or more of these capsid proteins and Rep proteins may be contained in the AAV genome. According to the present invention, the capsid protein (VP1, VP2 and/or VP3) and the Rep protein are preferably all encoded together by a single polynucleotide to be provided as an AAV helper plasmid.

A polynucleotide to be packaged in the AAV vector of the present invention (i.e., the polynucleotide) can be prepared by substituting the polynucleotide of an internal domain (i.e., either or both rep gene and cap gene) located between the ITRs on the 5' and 3' sides of the wild-type genome with a gene cassette containing a polynucleotide encoding the desired protein (the therapeutic gene), a promoter sequence for transcribing said polynucleotide, and the like. Preferably, the ITRs on the 3' and 5' sides are located at the 5' and 3' ends of the AAV genome, respectively. Preferably, the ITRs at the 5' and 3' ends of the rAAV genome of the present invention comprise the 5' ITR and the 3' ITR contained in the genome of AAV1, AAV2, AAV3, AAV4, AAV8, AAV9 or AAVrh10. Since the ITR parts generally have easily altered complementary sequences (flip and flop structure), the orientation of the 5' and 3' ITRs contained in the rAAV genome of the present invention may be inverted. In the rAAV genome of the present invention, the polynucleotide (namely, the therapeutic gene) that replaces the internal domain preferably has a practical length that is substantially equal to the length of the original polynucleotide. Specifically, the total length of the rAAV genome of the present invention is substantially equal to the total length of the wild-type, i.e., 5 kb, for example, about 2-6 kb, preferably about 4-6 kb. The length of the therapeutic gene incorporated into the rAAV genome of the present invention, excluding the length of the transcriptional regulatory region including the promoter, the polyadenylation site and the like (assuming, for example, about 1-1.5 kb), is preferably, but not limited to, about 0.01-3.7 kb, more preferably about 0.01-2.5 kb, and still more preferably 0.01-2 kb.

In general, if the polynucleotide (viral genome) packaged in the recombinant adeno-associated viral vector is a single strand, it may take time (several days) to express the desired therapeutic protein. In this case, the therapeutic gene to be transduced can be designed to take an sc (self-complementary) structure, so that it can exert its effect in a shorter period of time. Specific details are described, for example, in Foust K.D. et al., (Nat Biotechnol., 2009 Jan; 27(1): 59-65), etc. The polynucleotide packaged in the AAV vector of the present invention may have either a non-sc structure or an sc structure.

In one embodiment, the rAAV vector of the present invention preferably comprises a polynucleotide containing a promoter sequence specific for the cells in the central nervous system and a therapeutic gene operably linked to said promoter sequence (in other words, such a polynucleotide is packaged in a viral vector). The promoter sequence used in the present invention includes, but is not limited to, those derived from neurons, glial cells, oligodendrocytes, cerebral vascular endothelial cells, microglia, and cerebral ventricular epithelial cells. Examples of such promoter sequences specifically include, but are not limited to, synapsin I promoter sequence, glutamate decarboxylase (GAD65/GAD67) promoter sequence, Tie2 promoter sequence (brain capillary-specific promoter), myelin basic protein promoter sequence, neuron-specific enolase promoter sequence, glial fibrillary acidic protein promoter sequence, L7 promoter sequence (cerebellar Purkinje cell-specific promoter), glutamate receptor delta 2 promoter (cerebellar Purkinje cell-specific promoter), and oligodendrocyte-specific promoter (2',3'-cyclic nucleotide 3'-phosphodiesterase (CNP) promoter) sequence. In the rAAV vector of the present invention, promoter sequences such as WDR45 promoter (NM_001029896.2, NM_007075.4), NCOA4 promoter (NM_001145260.2, NM_001145261.2, NM_001145263.2, NM_001145262. 2, NM_005437.4), calcium/calmodulin-dependent protein kinase II (CMKII) promoter, αI-tubulin promoter, and platelet-derived growth factor β-chain promoter can also be used. The above promoter sequences may be used alone or in any combination.

Also, as the promoter sequence used in the present invention, powerful promoter sequences commonly used in the art, such as cytomegalovirus (CMV) promoter, CAG promoter, EF1-α promoter, and SV40 promoter, and enhancer sequences may also be used alone or in combination with the other promoter.

Examples of the particularly preferred promoter sequence used in the present invention include myelin basic protein promoter sequence, oligodendrocyte-specific promoter sequence, synapsin Ipromoter sequence, myelin basic protein promoter sequence, L7 promoter sequence (cerebellar Purkinje cell-specific promoter), glutamate receptor delta 2 promoter (cerebellar Purkinje cell-specific promoter), WDR45 promoter, NCOA4 promoter, CMV promoter, and CAG promoter.

Furthermore, sequences used in the rAAV vector of the present invention may include known sequences such as an enhancer sequence, an untranslated region sequence, Kozak sequence, and a suitable polyadenylation signal sequence, which can assist mRNA transcription, translation into protein, and the like.

The desired therapeutic gene, incorporated into the viral genome to be packaged in the rAAV vector of the present invention, is delivered to the nervous system cells in a highly efficient manner. The rAAV vector of the present invention is capable of gene transfer into about 10 times or greater, about 20 times or greater, about 30 times or greater, about 40 times or greater, or about 50 times or greater number of neurons than conventional rAAV vectors. The number of gene transferred neurons can be counted, for example, by creating an rAAV vector packaging an rAAV vector genome incorporating any marker gene, then administering the rAAV vector to a test animal, and counting the number of nervous system cells expressing the marker gene (or marker protein) incorporated in the rAAV vector genome. The marker gene used can be a known marker gene. Examples of such marker genes include LacZ gene, green fluorescent protein (GFP) gene, and luminescent protein gene (such as firefly luciferase).

### 5. Additional therapeutic gene

According to the present invention, as an additional means for increasing the expression level of NCOA4 protein in the target cells, for example, a means for suppressing the expression or function of the endogenous *de novo* mutant WDR45 protein can also be used. As such means, a vector or polynucleotide that targets the mutated portion of mutant WDR45 and cause gene disruption or reduced expression, such as an antisense molecule, a ribozyme, interfering RNA (iRNA), microRNA (miRNA), CRISPR-Cas9, etc., may be used. For example, to effectively inhibit the expression of a target gene using an antisense sequence, the length of the antisense nucleic acids is preferably 10 nucleotides or more, 15 nucleotides or more, or 20 nucleotides or more, preferably 100 nucleotides or more, and more preferably 500 nucleotides or more. Usually, the length of the antisense nucleic acids is shorter than 5 kb, preferably shorter than 2.5 kb. A ribozyme of interest can be designed by referring to various known literature (see, for example, FEBS Lett. 228: 228, 1988; FEBS Lett. 239: 285, 1988; Nucl. Acids. Res. 17: 7059, 1989; Nature 323: 349, 1986, etc.).

"RNAi" refers to the phenomenon in which the expressions of both introduced foreign gene and target endogenous gene are suppressed when double-stranded RNA having a sequence identical or similar to the target gene sequence is introduced into a cell. RNA used herein is, for example, a double-stranded RNA of 21-25 nucleotides in length that causes RNA interference, such as dsRNA (double-stranded RNA), siRNA (small interfering RNA), shRNA (short hairpin RNA), or miRNA (microRNA). Such RNA can be delivered locally to the desired site using a delivery system such as a liposome, or it can be expressed locally using a vector to generate the double-stranded RNA described above. Methods for preparing and using such double-stranded RNA (dsRNA, siRNA, shRNA, or miRNA) are known from many literature (see, JP-A-2002-516062; US 2002/086356A; Nature Genetics, 24(2), 180-183, 2000 Feb., etc.).

To use these multiple therapeutic genes, for example, a known internal ribosome entry site (IRES) sequence can be inserted into the polynucleotide carried by the vector of the present invention. If the rAAV genome has a non-sc structure, the promoter length and the target gene can be selected from a wider range, and it is possible to use multiple genes of interest. The total length of the polynucleotide packaged in the rAAV vector of the present invention is preferably about 5 kb or less (about 4.7 kb or less excluding the ITR region).

### 6. Preparation of rAAV vector of the present invention

A general method can be employed as the method for preparing the rAAV vector of the present invention. For example, the method may include a step of transfecting (a) a first polynucleotide encoding a capsid protein (generally referred to as an AAV helper plasmid), and (b) a second polynucleotide (containing a desired therapeutic gene) to be packaged in the rAAV vector of the present invention into cultured cells. The preparation method of the present invention may further include a step of transfecting (c) a plasmid encoding an adenovirus-derived factor, referred to as an adenovirus (AdV) helper plasmid, into cultured cells, or a step of infecting cultured cells with the adenovirus. The method can further include the steps of culturing the above transfected cultured cells and collecting the recombinant adeno-associated virus vector from the culture supernatant. Such a method is already known and is employed in the examples herein.

In the first polynucleotide (a), the nucleotides encoding the capsid protein of the present invention are preferably operably linked to a known promoter sequence that is operable in the cultured cells. As such a promoter sequence, for example, cytomegalovirus (CMV) promoter, EF-1α promoter, SV40 promoter, or the like may be used appropriately. Known enhancer sequence, untranslated region (UTR) sequence, Kozak sequence, polyA-addition signal sequences, etc. can also be contained appropriately.

The second polynucleotide (b) contains a therapeutic gene at a position operable with a promoter sequence such as the nervous system cell-specific promoter described above. Known enhancer sequence, Kozak sequence, polyA-addition signal sequences, etc. can also be contained appropriately. This first polynucleotide may also contain, downstream of the nervous system cell-specific promoter sequence, a cloning site that can be cleaved by various known restriction enzymes. A multiple cloning site containing multiple restriction enzyme recognition sites is more preferred. Those skilled in the art can incorporate a desired therapeutic gene downstream of the nervous system cell-specific promoter by following a known genetic engineering procedure. For such a genetic engineering procedure, see, for example, Molecular Cloning (Molecular Cloning 3rd Edition, J. Sambrook et al., Cold Spring Harbor Lab. Press. 2001), and the like.

In the preparation of the rAAV vector of the invention, a helper virus plasmid (e.g., adenovirus, herpesvirus, or vaccinia) may be used and introduced into the cultured cells simultaneously with the first and second polynucleotides described above. Preferably, the preparation method of the present invention further includes a step of introducing an adenovirus (AdV) helper plasmid. According to the present invention, the AdV helper is preferably derived from the same species of virus as the cultured cells. For example, if 293T human cultured cells are used, a helper virus vector derived from human AdV can be used. Such AdV helper vector may be a commercially available AdV helper vector (e.g., AAV Helper-Free System (catalog no. 240071) provided by Agilent Technologies).

In the preparation of the rAAV vector of the present invention, various known methods such as calcium phosphate method, lipofection method, and electroporation method can be used as the method for transfecting one or more of the plasmids above into cultured cells. Such methods are described, for example, in Molecular Cloning 3rd Ed., Current Protocols in Molecular Biology, John Wiley and Sons 1987-1997, and the like.

### 7. Pharmaceutical composition containing rAAV vector of the present invention

The rAAV vector of the present invention can contain a gene that is useful for the treatment of an iron metabolism disease (e.g., iron-accumulating neurodegenerative disease, etc.) caused by WDR45 and NCOA4 dysfunction. When the vector of the present invention containing these genes is administered, for example, intravascularly, it can pass through the blood-brain barrier to be incorporated into neurons in the brain, spinal cord, etc. Such rAAV vectors containing a therapeutic gene are included in the pharmaceutical composition of the present invention. The administration of such an rAAV vector to patients is expected to treat a neurological disease associated with an iron metabolic disorder.

While an active ingredient of the pharmaceutical composition of the present invention may be added alone or in combination, a pharmaceutically acceptable carrier or additive for formulation may be added thereto to provide the active ingredient in a form of a formulation. In this case, the content of the active ingredient of the present invention in the formulation may be, for example, 0.1-99.9% by weight.

The pharmaceutically acceptable carrier or additive, for example, an excipient, a disintegrant, a disintegration aid, a binder, a lubricant, a coating agent, a colorant, a diluent, a dissolution agent, a dissolution aid, a tonicity-adjusting agent, a pH regulator, a stabilizer, and the like may be used. For oral administration, the active ingredient, such as microcrystalline cellulose, sodium citrate, or calcium carbonate, starch, a disintegrant such as alginic acid, a granule-forming binder such as polyvinylpyrrolidone, a lubricant, and the like, can be used with excipients that are commonly used in the art

Examples of the formulation suitable for parenteral administration may include an injection, an intrathecal injection solution, and a suppository. For parenteral administration, a solution obtained by dissolving the active ingredient of the present invention in either sesame oil or peanut oil or in an aqueous propylene glycol solution can be used. The aqueous solution must first be appropriately buffered as needed (preferably, pH 8 or higher) so that the liquid diluent is isotonic. For example, physiological saline can be used as such a liquid diluent. The prepared aqueous solution is suitable for intravenous injection whereas the oily solutions are suitable for intra-articular injection, intramuscular injection, and subcutaneous injection.

In addition, if an aqueous suspension and/or elixir is desired for oral administration, the active ingredient may be used in combination with any sweetener or flavoring agent, a coloring agent or dye, and, if necessary, an emulsifier and/or suspending agent, together with a diluent such as water, ethanol, propylene glycol, glycerin, or a combination thereof. Examples of the formulation suitable for oral administration include powder, a tablet, a capsule, fine granules, granules, a liquid agent, and a syrup. All of these solutions can be produced readily under aseptic conditions by standard pharmaceutical techniques well known to those skilled in the art.

The dose of the pharmaceutical composition of the present invention is not particularly limited, and a suitable dose can be selected depending on various conditions including the type of the disease, age and symptoms of the patient, the administration route, the therapeutic goal, the presence of medicine used in combination therewith, and the like. A daily dose of the pharmaceutical composition of the present invention is, for example, but not limited to, 1-5,000 mg, preferably 10-1,000 mg, per adult (e.g., body weight 60 kg). The daily dose may be given in two to four divided doses. In terms of vg (vector genome), the dosage unit can be selected from, but is not limited to, a range of 10⁹-10¹⁴ vg, preferably 10¹⁰-10¹³ vg, and more preferably 10¹⁰-10¹² vg per kg body weight.

### 8. Administration of rAAV vector of the present invention

Since the rAAV of the present invention can pass through the blood-brain barrier of a living body (including an immature blood-brain barrier of a fetus and newborn and a mature blood-brain barrier of an adult), peripheral administration of the rAAV of the present invention to a living body (including an adult, fetus or newborn) allows for gene delivery to nervous system cells in the brain, spinal cord, etc. Furthermore, by peripheral administration, the rAAV vector used in the present invention can target neurons in the brain, spinal cord, etc. of an adult. As used herein, peripheral administration refers to an administration route generally recognized as peripheral administration by those skilled in the art, such as intravenous administration, intra-arterial administration, intraperitoneal administration, intracardiac administration, intramuscular administration, and intra-umbilical administration (for example, when targeting a fetus). In addition, a method of administration via fluid connected to the brain (other than blood), such as intrathecal administration, can also be used for the rAAV vector of the present invention. In another embodiment, the rAAV vector of the present invention can also be administered topically to a target site in the brain, such as the striatum and hippocampus. For example, the rAAV of the present invention can be administered into the spinal fluid by intrathecal administration, into the cisterna magna by insertion of a fine catheter into the medullary cavity, or into the blood by peripheral administration.

### 9. Kit for preparing rAAV vector of the present invention

According to another embodiment of the present invention, a kit for preparing rAAV of the present invention is provided. Such a kit comprises, for example, (a) a first polynucleotide for expressing a capsid protein VP1, etc., and (b) a second polynucleotide to be packaged in the rAAV vector. The first polynucleotide comprises, for example, a polynucleotide coding for the amino acids represented by SEQ ID NO: . The second polynucleotide, for example, may or may not contain a desired therapeutic gene, but preferably contains various restriction enzyme cleavage sites for incorporating such a desired therapeutic gene.

The kit for preparing a rAAV vector of the present invention may further comprise any component described herein (e.g., AdV helper, etc.). In addition, the kit of the present invention may further comprise instructions describing the protocol for preparing a rAAV vector using the kit of the present invention.

### 10. Measurement of the therapeutic effects of rAAV of the present invention

### (a) Measuring iron metabolic capacity of living body

The therapeutic effects of the rAAV vector of the present invention can be determined by monitoring the restoration of iron metabolism and the reduction of iron accumulation in the brain over time in a patient (or animal) administered with the vector of the present invention, for example, using MRI.

To evaluate iron accumulation in the brain, brain MRI is the easiest and most sensitive method to detect iron in the central nervous system and is also suitable for evaluation over time.

In addition to, or as an alternative to, the above evaluation by brain MRI or the like, it is also possible to determine the therapeutic effect by a more comprehensive evaluation for example, the motor capacity, etc. of a living body (Barthel Index: MAHONEY FI, BARTHEL DW. FUNCTIONAL EVALUATION: THE BARTHEL INDEX. Md State Med J. 1965; 14: 61-5; The Unified Parkinson's Disease Rating Scale (UPDRS): Fahn S, Marsden CD, Calne DB, Goldstein M, ed., Recent Developments in Parkinson's Disease, Vol 2. Florham Park, NJ. Macmillan Health Care Information 1987, 153-163, 293-304).

Various *in vitro* means known in the art may also be used.

For example, the iron metabolic capacity of a living body can be estimated by measuring serum ferritin and serum iron, which are general serum biochemical tests, and it can be determined by measuring serum ferritin and serum iron in the living body over time.

When cells obtained from a living body are used, the cell type used may usually be dermal fibroblasts or lymphoid cells. In some cases, cells that have differentiated from pluripotent stem cells can be used.

For tissue and cell staining, a known ferric iron staining substance such as Berlin Blue stain (Prussian Blue stain) (reference URL: https://labchem-wako.fujifilm.com/jp/product/detail/W01W0129-2154.html), and/or a ferrous iron staining substance such as FerroOrange stain (https://www.dojindo.co.jp/products/F374/?utm_source=top &utm_medium=referral) can be used for coloration.

### (b) Measuring iron metabolic capacity in vitro or ex vivo

The present invention also provides a method for identifying cells derived from patients with an iron-accumulating neurodegenerative disease (or individuals at risk thereof) by comparing the expression level of NCOA4 (protein or nucleotide) in a cell sample with that in control cells derived from a healthy individual. According to this method, the cell sample is preferably one derived from a patient. The cell sample may also be obtained by growing cells derived from a patient *in vitro* in culture. Examples of a preferred cell types as a cell sample include, but are not limited to, cells derived from oligodendrocytes, neurons, astrocytes, glial cells, lymphoid cells, and dermal fibroblasts.

In detecting the iron metabolic capacity *in vitro* or *ex vivo,* cells such as oligodendrocytes, neurons, astrocytes, glial cells, lymphoid cells, dermal fibroblasts or the like obtained from a patient (or animal) administered with the rAAV vector of the present invention may be used as a sample.

In detecting the iron metabolic capacity *in vitro* or *ex vivo,* a method may be used, in which, instead of NCOA4 or in addition to NCOA4, the expression level of ferroportin, the expression level of ferritin heavy chain, the expression level of ferritin light chain, the expression level of DMT1, the expression level of transferrin receptor, and/or the expression level of hepcidin in the above cells is compared with their expression levels in control cells obtained from a healthy individual (e.g., a healthy person).

### (c) Evaluation of iron metabolic capacity

According to the present invention, regulation of the intracellular iron metabolism refers to the ability to improve or reduce the iron metabolic capacity in cell samples such as cells derived from a living body or cultured cells with reduced iron metabolic capacity, and preferably refers to the ability to increase the activity involved in iron metabolism. Although ferrous iron has toxic effects such as the production of reactive oxygen species, a deficiency of ferrous iron also has adverse effects on the living body, such as reduced mitochondrial function. Therefore strict regulation is required. On the other hand, ferric iron, which is produced by oxidation of ferrous iron, is stored in ferritin and becomes nontoxic.

Herein, evaluation of iron metabolic capacity include, for example, measurements of intracellular iron ion content (ferrous iron and ferric iron) and ferritin content, and the capacity of stored iron degradation.

The intracellular iron ion content is measured by measuring the total amount of ferrous and ferric iron ions, or by indirectly measuring the amount of ferric iron by measuring ferritin.

As a means of measuring the iron ion content (ferrous iron, ferric iron), for example, a means known in the art can be used, such as an iron ion measurement kit (Sigma MAK025 https://www.sigmaaldrich.com/JP/ja/product/sigma/mak025), or the like.

For the measurement of intracellular ferric iron, the ferric iron content can be quantified based on the color intensity obtained, for example, by Berlin Blue staining (or Prussian Blue staining; https://labchem-wako.fujifilm.com/jp/product/detail/W01W0129-2154.html), or the like.

For the measurement of intracellular ferrous iron, the ferrous iron content can be quantified based on the color intensity obtained, for example, by FerroOrange staining (https://www.dojindo.co.jp/products/F374/?utm_source=top&utm_medium=referral), or the like.

For example, according to the present invention, as a result of the regulation of the iron metabolism in a living body or in cells derived from that living body administered with the rAAV vector of the present invention, the iron metabolic capacity (or the indicator of iron accumulation) based on the results obtained by the means above is in the range of 50-200%, preferably 55% or more, 60% or more, 65% or more, 70% or more, 75% or more, 80% or more, 85% or more, 90% or more, or 95% or more, and 200% or less, 190% or less, 180% or less, 170% or less, 160% or less, 150% or less, 140% or less, 130% or less, 120% or less, 110% or less, or 100% or less, compared to the capacity of the healthy individuals or control cells derived from the healthy individuals.

Quantification of each iron ion by staining may only be an approximate indicator.

In addition, a degradation ability for iron can be determined from the increase or decrease in ferritin content compared to a control. For the measurement of intracellular ferritin content, a known means such as Western blotting or quantitative RT-PCR (qRT-PCR) can be used. Herein, the increase or decrease in the ferritin content obtained is assumed to be directly related to a degradation ability for stored iron, and a decrease in the ferritin content is considered to indicate the progress of iron degradation (Figure 1). The ferritin content in this case may be the ferritin heavy chain content, the ferritin light chain content, or the sum of both. Moreover, the expression level of ferritin heavy chain can be measured based on the oxidation of ferrous iron to ferric iron.

Other available means of detecting the above degradation ability for the stored iron include, but are not limited to, NCOA1 expression level, ferroportin expression level, DMT1 expression level, transferrin receptor expression level, and hepcidin expression level in the sample cells. As a means of measuring the expression levels of NCOA4, ferroportin, DMT1, transferrin receptor, hepcidin, and the like mentioned above, any means known to those skilled in the art, such as Western blotting using an antibody against each protein, and RT-PCR and Northern blotting for mRNA of each protein, can be used. Herein, the increase or decrease in the expression levels of NCOA4, ferroportin, DMT1, transferrin receptor, and/or hepcidin is assumed to be related to the degradation ability for the stored iron.

According to the present invention, restoration of the expression level of the protein of interest in the cells used (e.g., cells derived from patients, cells derived from model animals, etc.) means that the expression level detected in terms of the protein level is in the range of about 50-200%, preferably 55% or more, 60% or more, 65% or more, 70% or more, 75% or more, 80% or more, 85% or more, 90% or more, or 95% or more, and 200% or less, 190% or less, 180% or less, 170% or less, 160% or less, 150% or less, 140% or less, 130% or less, 120% or less, 110% or less, or 100% or less, compared to the expression level in normal control cells (e.g., cells derived from healthy individuals). As a means of detecting the expression level of proteins, methods known in the art may be used, such as immunoblotting (e.g., Western blotting), color intensity obtained by direct staining of the protein component (e.g., Coomassie staining), or quantitative RT-PCR.

The patient, from whom the cells identified by the above method have been derived, may be further evaluated for iron accumulation using the above means of evaluation, such as MRI or the like, to confirm iron accumulation in the brain, motor abilities, and the like.

### (d) Measuring autophagy activity

To measure the iron metabolic capacity of cells according to the present invention, the autophagy activity or the expression level of an autophagy-related factor in the cells can be determined to indirectly predict, determine and/or evaluate iron accumulation in a living body. Examples of such an autophagy-related factor include LC3-II, and the increase or decrease in LC3-II can be determined as an indicator of autophagy activity, for example, by using Flux-assay (https://proteolysis.jp/autophagy/protocol/protocol%20files/LC3%20flux% 20assay.pdf).

Alternatively, a fluorescent probe (GFP-LC3-RFP) for measuring autophagy activity may be transferred into target cells using retrovirus to evaluate the degradation efficiency by the GFP/RFP ratio (Kaizuka, T. et al., (2016). Mol Cell 64(4): 835-849). More specifically, the GFP/RFP ratio after incubation for a certain period of time under an autophagy induction condition (amino acid starvation condition) or an inhibition condition (addition of Bafilomycin A1) can be evaluated, where the difference between the induction condition and the inhibition condition allows comparison of autophagy activity between the cells.

According to the present invention, when the autophagy capacity is judged to be restored to normal level by the above procedure, the activity or expression level obtained from the sample of interest (cells derived from patients, cells derived from model animals, etc.) is in the range of about 50-200%, preferably 55% or more, 60% or more, 65% or more, 70% or more, 75% or more, 80% or more, 85% or more, 90% or more, or 95% or more, and 200% or less, 190% or less, 180% or less, 170% or less, 160% or less, 150% or less, 140% or less, 130% or less, 120% or less, 110% or less, or 100% or less, compared to that of the control.

### 11. Methods for screening substance for treating NCOA4-related diseases

The present invention also provides methods for screening substances for restoring iron metabolism. This screening method is, for example, a method for obtaining a candidate substance that increases the expression level of NCOA4 (protein or polynucleotide). This method may comprise the steps of: contacting a test substance with a target cell (e.g., a patient-derived cell); measuring an expression level of NCOA4 in the target cell in the presence of the test substance; comparing the obtained expression level of NCOA4 with an expression level of NCOA4 in the target cell in the absence of the test substance; and obtaining a candidate substance that increases the expression level of NCOA4 in the target cell. The method may also include a step of comparing the expression level of NCOA4 with that in a control cell (e.g., cells derived from healthy individuals).

As the above test substance, a substance that acts on the promoter of NCOA4 may be used. Various substances known in the art, for example, a low molecular weight compound and an organic substance such as a peptide, a protein or a lipid can be used as such a substance. In some cases, reference can be made to the sequence of the promoter region of NCOA4.

In the screening method above, cell samples derived from patients include, but are not limited to, oligodendrocytes, neurons, astrocytes, glial cells, lymphoid cells, and dermal fibroblasts for example.

The above method may further include a step of comparing the expression level of ferroportin, the expression level of ferritin heavy chain, and/or the expression level of ferritin light chain in the above cells with these expression levels in healthy control cells. The expression levels of these substances can be measured using an antibody against each substance, RT-PCR, or other means known to those skilled in the art.

The method may further comprise a step of applying the candidate substance obtained as the result of screening in the above method to a model cell or a model animal with an iron-accumulating neurodegenerative disease to verify the candidate substance's capacity related to iron metabolism. For this verification, the above-described procedure for assessing iron accumulation capacity can be used.

### 12. Terms used in this specification

The meaning of each term used herein is as follows. For the terms that are not specifically explained herein are intended to have the meanings within the range generally understood by those skilled in the art.

As used herein, unless otherwise stated, the terms "viral vector", "virus particle", and "viral virion" are used interchangeably.

As used herein, the term "nervous system" refers to an organ system composed of nervous tissue. As used herein, the term "nervous system cells" includes at least neurons in the central nervous system such as the brain and spinal cord and may also include glial cells, microglia, astrocytes, oligodendrocytes, cerebral ventricular ependymal cells, cerebral vascular endothelial cells, and the like.

As used herein, the term "polynucleotide" is used interchangeably with "nucleic acids", "gene" or "nucleic acid molecule", and is intended to refer to a polymer of nucleotides. As used herein, the term "nucleotide sequence" is used interchangeably with "nucleic acid sequence" or "base sequence" and is represented by a sequence of deoxyribonucleotides (abbreviated as A, G, C, and T). For example, a "polynucleotide comprising the nucleotide sequence represented by SEQ ID NO: 1 or a fragment thereof' is intended to refer to a polynucleotide comprising the sequence represented by the respective deoxynucleotides A, G, C and/or T of SEQ ID NO: 1, or a fragment thereof.

The "viral genome" and "polynucleotide" according to the present invention may exist in a form of DNA (e.g., cDNA or genomic DNA) or, in some cases, in a form of RNA (e.g., mRNA). The viral genome and the polynucleotide as used herein may be a double-stranded or single-stranded DNA. In the case of a single-stranded DNA or RNA, it may be a coding strand (also known as a sense strand) or a non-coding strand (also known as an antisense strand). Unless otherwise specified, when a promoter, a gene of interest, a polyadenylation signal, and the others encoded by the rAAV genome are described herein with respect to their locations in the gene, the strand itself is described if the rAAV genome is a sense strand and its complementary strand is described if it is an antisense strand.

As used herein, the terms "protein" and "polypeptide" are used interchangeably and intended to refer to a polymer of amino acids. A polypeptide as used herein is represented in accordance with a conventional peptide designation, in which the N-terminal (amino terminal) is shown on the left and the C-terminal (carboxyl terminal) on the right. A partial peptide of the polypeptide of the present invention (which, may be simply referred to herein as a "partial peptide of the present invention") includes a partial peptide of the polypeptide of the present invention described above, and preferably has the same property as said polypeptide of the present invention.

As used herein, the term "plasmid" refers to any of various known gene elements, for example, a plasmid, a phage, a transposon, a cosmid, a chromosome, etc. A plasmid can replicate in a specific host and can transport a gene sequence between cells. As used herein, a plasmid includes various known nucleotides (DNA, RNA, PNA, and a mixture thereof) and may be a single strand or a double strand, preferably a double strand. For example, as used herein, unless otherwise specified, the term "rAAV vector plasmid" is intended to include a double strand formed of a rAAV vector genome and its complementary strand. The plasmid used in the present invention may be either linear or cyclic.

As used herein, the term "packaging" refers to events including preparation of a single-stranded viral genome, assembly of a coat (capsid) protein, enclosure of the viral genome within the capsid (encapsidation), etc. When an appropriate plasmid vector (generally, multiple plasmids) is introduced into cell lines that allows packaging under appropriate conditions, recombinant viral particles (i.e., virus virions, virus vectors) are assembled and secreted into the culture.

### EXAMPLES

Hereinafter, the present invention will be described more specifically by means of examples, although the scope of the present invention should not be limited to the following examples.

### (1) Cell culture

Human dermal fibroblasts were cultured using Dulbecco's modified Eagle's medium (Gibco, 11885) with 10% fetal bovine serum (Gibco), and 1% streptomycin/penicillin (Gibco, 15140) under the conditions of 37°C, 100% humidity, and 5% CO₂ concentration.

### (2) Western blot

Cell lysis buffer MPER (Thermo Fisher, 78503) containing protease inhibitor (Roche, 11873580001) and phosphatase inhibitor (Roche, 04906845001) was used for protein extraction. After aspirating the medium from the cell culture dish, cells were washed twice with PBS, and incubated with the cell lysis buffer on ice for 5 minutes. Then, the cells were collected in a tube with a cell scraper. After vortexing, the sample was centrifuged at 4°C, 14,000 x g, for 10 minutes, and the supernatant was collected in a new tube.

Equal amounts of protein were separated by SDS-PAGE on 4-12% gels (Thermo Fisher, WG1403BX10) and transferred to a PVDF membrane (Thermo Fisher, IB24001) using iBlot2 (Thermo Fisher). After transfer, the membrane was blocked in a blocking buffer (5% skim milk in PBS-T) at room temperature for 1 hour. After washing with PBS-T, the cells were incubated overnight at 4°C in an antibody diluent (1% skim milk in PBS-T) added with a primary antibody. After washing, the cells were incubated in an antibody diluent containing an HRP-labeled secondary antibody at room temperature for 1 hour. After washing, the cells were detected and analyzed by Amersham Imager (GE Healthcare) using the HRP detection reagent (Takara, T7103A or Thermo Fisher, A38554).

**[Table 1]**

| Antibodies used for Western blotting | | | |
|---|---|---|---|
| Antibody | Manufacturer | Product No. | Dilution |
| Rabbit polyclonal anti-WDR45 antibody | Proteintech | 19194-1-AP | 1:500 |
| Mouse monoclonal anti-ferritin heavy chain antibody | Santa Cruz | sc-386594 | 1:5000 |
| Mouse monoclonal anti-ferritin light chain antibody | Santa Cruz | sc-74513 | 1:5000 |
| Rabbit polyclonal anti-NCOA4 antibody | Thermo Fisher | A302-0272A | 1:500 |
| Mouse monoclonal anti-transferrin receptor antibody | Thermo Fisher | 13-6800 | 1:5000 |
| Mouse monoclonal anti-DMT 1 antibody | Abnova | H00004891-MO1 | 1:1000 |
| Rabbit polyclonal anti-ferroportin antibody | ALPHA DIAGNOSIS | MTP11-A | 1:5000 |
| HRP-conjugated monoclonal anti-β-actin antibody | MBL | PM053-7 | 1:5000 |
| Goat anti-mouse IgG antibody | Thermo Fisher | G-21234 | 1:5000 |
| Goat anti-rabbit IgG antibody | Thermo Fisher | G-21040 | 1:5000 |

### (3) Quantitative real-time PCR (qRT-PCT)

Total RNA was extracted from the fibroblasts using TRIzol Reagent (Thermo Fisher, 15596018). Quantification was performed using NanoDrop 8000 microvolume spectrophotometer (Thermo Fisher). SuperScript IV VILO Master Mix (Thermo Fisher, 11756500) was used to synthesize cDNA with 300-700 ng of RNA according to the manufacturer's instructions. A total of 20 µL per well of PCR reaction solution was prepared with 10 µL of TaqMan Gene Expression Master Mix (Thermo Fisher, 4369016), 1 µL each of TaqMan Gene Expression Assay (see table for details of the probe), 1 µL of synthesized cDNA, and 7 µL of RNase-free water (Takara, 9012). QuantStrdio 3 (Thermo Fisher) was used for the measurement. PCR reaction conditions were as follows: 50°C for 2 minutes and 95°C for 10 minutes, followed by 40 cycles of 95°C for 15 seconds and 60°C for 1 minute, and then Melt Curve was determined. Relative quantification was performed using QuantStudio Design and Analysis Software according to ΔΔCt method using GAPDH as the internal control.

**[Table 2]**

| TaqMan probes used for quantitative real-time PCR | |
|---|---|
| GAPDH | Hs03929097_g1 |
| WDR45 | Hs01079049_g1 |

| | |
|---|---|
| (Nucleotide sequence of each primer is not publicly available.) | |

### (4) AAV.GTX-CMV-WDR45-WPRE Vector

The vector used in this example (hereinafter also referred to as "AAV.GTX") was obtained by inserting an expression cassette consisting of human cytomegalovirus immediate-early (CMV) promoter, cDNA of WDR45 (NM_007075.4), and simian virus 40 polyadenylation signal sequence into AAV9/3 (a viral vector having a tyrosine mutation (Y446 -> F) in the capsid of AAV9 (SEQ ID NO: 9) and an ITR sequence derived from AAV3), as described in WO2012/057363.

### (5) Gene transfer of WDR45 with AAV

The fibroblasts were seeded to reach 20-30% confluence the next day, and the medium was replaced the next day (day 0) with a medium supplemented with AAV.GTX-CMV-WDR45-WPRE at 5 x 10⁵ vg/cell. The next day (day 1), the same volume of medium as the day before was added. The next day (day 3), cells were used for downstream analysis.

### (6) Iron detection

Sample cells were lysed to extract proteins to perform SDS-PAGE and Western blotting to determine whether the cells were normalized in terms of an increase or decrease in the iron-related factors compared to cells derived from a healthy individual.

Alternatively, RNAs were extracted from the cells and subjected to reverse transcription to produce cDNAs, and normalization was determined by an increase or decrease in gene expression by qRT-PCR compared to cells derived from a healthy individual.

Alternatively, Berlin Blue staining (or Prussian Blue staining; https://labchem-wako.fujifilm.com/jp/product/detail/WO1WO129-2154.html) was used to stain ferric iron contained within ferritin, and FerroOrange staining was used to stain ferrous iron. More specific methods will be described below.

### (a) Berlin Blue staining (modified)

The fibroblasts were cultured on a coverslip (Matsunami Glass Ind., Ltd., C012001) to 70-80% confluence and fixed with 4% PFA at room temperature for 15 minutes. After washing with PBS, 50 µg/mL digitonin (Wako, 043-21376) in PBS was added to cause perforation at room temperature for 5 minutes. After washing with PBS, a mixture of 2% potassium ferrocyanide and 2% hydrogen chloride (Wako, 25985-50) was added and reacted at room temperature for 6 hours. After washing with pure water, Metal Enhancer for DAB stain (nacalai, 07388-24) mixed with Peroxidase Stain DAB kit (nacalai, 25985-50) was added and reacted at room temperature for 2 hours. After dehydration with ethanol and xylene, the coverslips were sealed in glass slides with balsam.

### (b) Evaluation of ferrous iron with FerroOrange (method using coverslip)

The fibroblasts were cultured on a coverslip to 70-80% confluence, to which a culture medium containing 1 µM FerroOrange (DOJINDO, F374) was added and incubated at 37°C and 5% CO₂ concentration for 30 minutes. The medium was aspirated and fixed with 4% PFA at room temperature for 15 minutes. After washing with PBS, the coverslips were sealed in a glass slide with ProLong Glass (ThermoFisher, P36980). Fluorescence was observed with BZ-X810 fluorescence microscope (KEYENCE) using TRITC and DAPI filters. The integrated brightness of FerroOrange was divided by the number of nuclei to determine the relative content of ferrous iron in each sample.

### (c) Evaluation of ferrous iron with FerroOrange (method using 96-well plate)

The fibroblasts were seeded into 96-well plates and cultured to 90% confluence. The cells were washed twice with PBS, and 80 µL/well of HBSS (Wako, 085-09355) supplemented with 1 µM FerroOrange (DOJINDO, F374) and 1 µg/mL Hoechst 33342

(DOJINDO, 346-07951) was added. The resultant was incubated at 37°C and 5% CO₂ for 30 minutes and fluorescence was measured using EnVision plate reader (Perkin Elmer). Filters were used so that the excitation and emission wavelengths of FerroOrange were 550 nm and 620 nm, respectively, and the excitation and emission wavelengths of Hoechst were 355 nm and 460 nm, respectively. The relative ferrous iron content in each sample was evaluated using the blank-corrected FerroOrange/Hoechst ratio.

### (7) Administration of vector of the present invention to iron-accumulating animal model

The vector of the present invention was administered to a subject, for example, via a direct route, i.e., to the central nervous system by inserting a fine catheter into the subject's medullary cavity and allowing the apex to reach the cisterna magna, and/or by transvenous systemic administration.

After administration, the effect of the introduction of the vector of the present invention was verified by monitoring states of iron accumulation by MRI measurement in the brain of the living body, or by measuring the expression levels of WDR45, ferritin heavy chain, ferritin light chain, NCOA4, transferrin receptor, DMT1, ferroportin, and/or LC3 in sample cells (lymphoid cells, dermal fibroblasts, etc.) over time.

### Experimental results

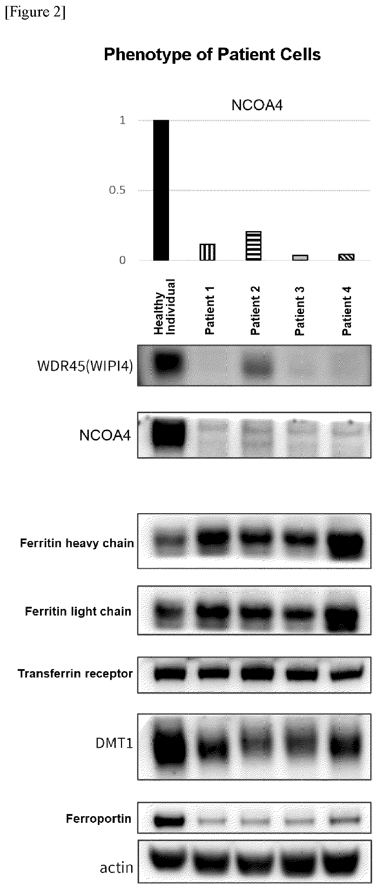

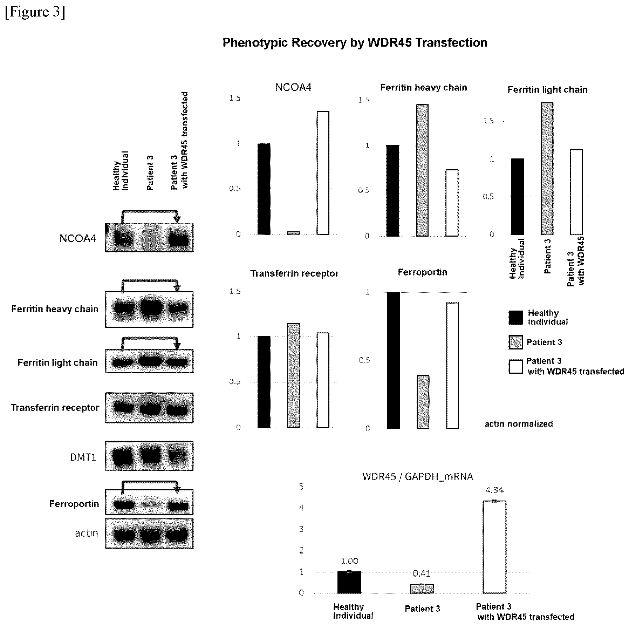

Western blotting of the cells obtained from Patients 1-4 confirmed significant decreases in the expression level of WDR45 and significant decreases in NCOA4 (approximately 20% or less) compared to the cells from a healthy individual (Figures 2 and 3).

Furthermore, when WDR45 was expressed by external gene transfer in the cells obtained from Patient 3, the expression levels of NCOA4 and ferroportin were restored, and the expression levels of ferritin heavy chain and ferritin light chain were restored to normal levels (Figure 3).

These changes in the expression levels indicate that the excessively accumulated iron (ferric iron) stores in the cells were degraded and became available as ferrous iron, which is essential for cell survival, and that ferrous iron could be supplied from the iron stores to the intracellular organelles.

### INDUSTRIAL APPLICABILITY

The rAAV vector of the present invention is expected to treat (alleviate, ameliorate, recovery, etc.) iron-accumulating neurodegenerative diseases. Iron accumulation in the central nervous system is observed not only in NBIA, but also in neurodegenerative diseases such as Alzheimer's disease, Parkinson's disease, and amyotrophic lateral sclerosis. Therefore, if iron accumulation in the central nervous system can be reduced by normalizing iron metabolism with the vector of the present invention, the present invention is also expected to be applicable to the treatment of these diseases.

### SEQUENCE LISTING FREE TEXT

SEQ ID NO: 1: Amino acid sequence of human WDR45 (isoform 1) (NP_009006.2) (in which the first Met is excluded)
SEQ ID NO: 2: Amino acid sequence of human WDR45 (isoform 2) (NP_001025067.1) (in which the first Met is excluded)
SEQ ID NO: 3: Amino acid sequence of human NCOA4 (650aa) (isoform 1) (NM_001145260.2)
SEQ ID NO: 4: Amino acid sequence of human NCOA4 (630aa) (isoform 2) (NP_001138733.1)
SEQ ID NO: 5: Amino acid sequence of human NCOA4 (614aa) (isoform 3) (AAH01562.1, NP_001138734.1, NP_001138735.1, NP_005428.1)
SEQ ID NO: 6: Amino acid sequence of human NCOA4 (575aa) (AAH12736.1)
SEQ ID NO: 7: Amino acid sequence in which tyrosine at position 445 in the amino acid sequence of VP1 capsid protein of wild-type AAV1 is replaced by phenylalanine (in which the first Met is excluded)
SEQ ID NO: 8: Amino acid sequence in which tyrosine at position 444 in the amino acid sequence of VP1 capsid protein of wild-type AAV2 is replaced by phenylalanine (in which the first Met is excluded)
SEQ ID NO: 9: Amino acid sequence in which the tyrosine residue at position 446 in the amino acid sequence of VP1 capsid protein of wild-type AAV9 is replaced by a phenylalanine residue (in which the first Met is excluded)

## Claims

1. A recombinant adeno-associated virus vector for use in intracellularly expressing any one of the amino acid sequences represented by SEQ ID NOs: 3-6, the recombinant adeno-associated virus vector comprising a polynucleotide encoding the amino acid sequence represented by SEQ ID NO: 1 or 2, or an amino acid sequence having about 90% or more identity to the amino acid sequence represented by SEQ ID NO: 1 or 2.

2. The adeno-associated virus vector according to claim 1, which is used to regulate intracellular autophagy capacity and/or iron metabolism capacity.

3. The adeno-associated virus vector according to claim 1, which is used for the treatment of an iron-accumulating neurodegenerative disease.

4. The adeno-associated virus vector according to claim 1, which is used for the treatment of SENDA/BPAN (WDR45 abnormality).

5. The recombinant adeno-associated virus vector according to claim 1, comprising a capsid protein of wild-type AAV1, AAV2, AAV9, or AAVrh10.

6. The recombinant adeno-associated virus vector according to claim 1, comprising a capsid protein having a mutant amino acid sequence in which tyrosine at position 445 in the amino acid sequence of wild-type AAV1 capsid protein is replaced with phenylalanine, a capsid protein having a mutant amino acid sequence in which tyrosine at position 445 in the amino acid sequence of wild-type AAV2 capsid protein is replaced with phenylalanine, or a capsid protein having a mutant amino acid sequence in which tyrosine at position 446 in the amino acid sequence of wild-type AAV9 capsid protein is replaced with phenylalanine.

7. The recombinant adeno-associated virus vector according to claim 1, wherein the polynucleotide comprises an inverted terminal repeat (ITR) selected from the group consisting of AAV1, AAV2, AAV3, AAV4, AAV8, and AAV9.

8. The recombinant adeno-associated virus vector according to claim 1, wherein the polynucleotide comprises a promoter sequence selected from the group consisting of synapsin I promoter sequence, myelin basic protein promoter sequence, neuron-specific enolase promoter sequence, calcium/calmodulin-dependent protein kinase II (CMKII) promoter sequence, αI-tubulin promoter sequence, platelet-derived growth factor β-chain promoter sequence, glial fibrillary acidic protein (GFAP) promoter sequence, L7 promoter sequence (cerebellar Purkinje cell-specific promoter), glial fibrillary acidic protein (hGfa2) promoter sequence, glutamate receptor delta 2 promoter (cerebellar Purkinje cell-specific promoter) sequence, glutamate decarboxylase (GAD65/GAD67) promoter sequence, WDR45 promoter, NCOA4 promoter, cytomegalovirus promoter, and CAG promoter.

9. The adeno-associated virus vector according to claim 1, further comprising a polynucleotide encoding any one of the amino acid sequences represented by SEQ ID NOs: 3-6 or an amino acid sequence having about 90% or more identity to any one of the amino acid sequences represented by SEQ ID NOs: 3-6.

10. A pharmaceutical composition comprising the recombinant adeno-associated virus vector according to any one of claims 1-9.

11. A pharmaceutical composition comprising the recombinant adeno-associated virus vector according to any one of claims 1-8, and a recombinant adeno-associated virus vector comprising a polynucleotide encoding any one of the amino acid sequences represented by SEQ ID NOs: 3-6 or an amino acid sequence having about 90% or more identity to any one of the amino acid sequences represented by SEQ ID NOs: 3-6.

12. The pharmaceutical composition according to claim 10, which is administered intracerebrally, intrathecally or peripherally.

13. A method for identifying, *in vitro* or *ex vivo,* a cell that causes an iron-accumulating neurodegenerative disease in an organism, the method comprising the steps of:
providing sample cells derived from the organism;
measuring the expression level of any one of the amino acid sequences represented by SEQ ID NOs: 3-6 or a coding sequence thereof in the sample cells; and
comparing the measured expression level with the expression level of said any one of the amino acid sequences represented by SEQ ID NOs: 3-6 or a coding sequence thereof in a control cell.

14. The method according to claim 13, wherein the sample cells are selected from the group consisting of oligodendrocytes, neurons, astrocytes, glial cells, lymphoid cells, and dermal fibroblasts.

15. The method according to claim 13, wherein the sample cells are cultured *in vitro.*

16. The method according to claim 13, comprising a step of measuring the expression of any one of the amino acid sequences represented by SEQ ID NOs: 3-6 or a coding sequence thereof with an antibody or by RT-PCR.
